Europäisches Patentamt

European Patent Office

Office européen des brevets

(11) Numéro de publication: **0 244 292**

**A1**

# DEMANDE DE BREVET EUROPEEN

(21) Numéro de dépôt: **87400843.6**

(22) Date de dépôt: **14.04.87**

(51) Int. Cl.4: **G 01 T 1/29**
G 01 T 1/164, G 01 T 5/08,
A 61 B 6/03

(30) Priorité: **18.04.86 FR 8605644**

(43) Date de publication de la demande:
**04.11.87 Bulletin 87/45**

(84) Etats contractants désignés:
**BE DE GB IT NL SE**

(71) Demandeur: **THOMSON-CSF**
**173, Boulevard Haussmann**
**F-75379 Paris Cédex 08 (FR)**

(72) Inventeur: **Micheron, François**
**THOMSON-CSF SCPI 19, avenue de Messine**
**F-75008 Paris (FR)**

**Staron, Alain**
**THOMSON-CSF SCPI 19, avenue de Messine**
**F-75008 Paris (FR)**

(74) Mandataire: **Lincot, Georges et al**
**THOMSON-CSF SCPI 19, avenue de Messine**
**F-75008 Paris (FR)**

(54) **Dispositif pour l'acquisition d'une image de contraste d'un objet placé dans un faisceau de radiations ionisantes.**

(57) Le dispositif comprend au moins un réseau plan de guides de lumière (2) émetteurs de photons sous l'effet des radiations, chaque réseau pouvant occuper un nombre déterminé d'orientations dans son plan.

Des capteurs photoélectriques (11) sont disposés en regard des extrémités des guides de lumière pour transmettre sous la forme de signaux électriques l'énergie lumineuse engendrée pour les guides de lumière (2).

Application : appareils d'imaagerrie en rayonnement ionisant.

FIG_1

EP 0 244 292 A1

## Description

Dispositif pour l'acquisition d'une image de contraste d'un objet placé dans un faisceau de radiations ionisantes

La présente invention concerne un dispositif pour l'acquisition d'une image de contraste d'un objet placé dans un faisceau de radiations ionisantes.

Elle s'applique plus particulièrement à la réalisation d'appareils d'imagerie en rayonnement ionisants.

Un procédé actuellement très utilisé pour obtenir une image par contraste d'un objet soumis à des radiations ionisantes consiste à former l'image sur un film radiographique. Outre le fait que le développement du film est d'une relative complexité, les images obtenues sont peu contrastées et la taille non négligeable des clichés (40 × 40 cm) rend le procédé coûteux. D'autre part, sa résolution est largement surdimensionnée pour les besoins de la radiothérapie, une erreur de 5 mm dans la position du champ à irradier se traduisant par une variation de 3% de la dose absorbée pour des photons de 1 Mev.

Certains laboratoires utilisent des prototypes de xéroradiographie, mais la dynamique obtenue n'est pas suffisante.

Quant à la fluoroscopie qui consiste à transformer sur un écran des photons de rayons X en photons visibles,

- d'une part, celle-ci suppose une place importante sous le lit du patient, pour loger un réducteur optique, d'où il résulte des difficultés de mise en oeuvre et des coûts d'installation élevés,

et d'autre part, celle-ci met en oeuvre une méthode de mesure qui demande un temps d'intégration important et qui ne tient pas compte du fait que lorsque les radiations ionisantes sont obtenues à partir d'un canon à électrons, comme en radiothérapie, le flux de photons est délivré sous forme de flashes brefs et intenses. Ceci se traduit par un éblouissement du dispositif et consécutivement par une perte de contraste.

Un perfectionnement à cette dernière technique est cependant connu par l'enseignement de la demande de brevet GB 2 061 055 A. Le dispositif qui y est décrit met en oeuvre un assemblage de bandes parallèles en matériau scintillant, disposées sur un même plan et mises en rotation autour de l'axe d'un faisceau de rayons X. Des capteurs photoélectriques disposés à une extrémité de chaque bande permettent de transmettre les signaux lumineux fournis par les bandes pendant la rotation. Cependant avec ce dispositif le corps du patient qui est interposé entre une source de rayons X et les bandes est irradié pendant tout le mouvement de rotation des bandes. Comme le mouvement dure pendant un temps relativement long d'environ 1 s il s'ensuit une irradiation prohibitive qui dépasse très largement la durée normale d'un examen habituellement fixée à 40 ms.

Le but de l'invention est de pallier les inconvénients cités.

A cet effet, l'invention a pour objet un dispositif pour l'acquisition d'une image de contraste d'un objet placé dans un faisceau de radiations ionisantes émis par une source de radiations, l'objet irradié étant interposé entre la source de radiations et le dispositif, comprenant au moins un réseau de guides de lumière émetteurs de photons sous l'effet des radiations, les guides de lumière d'un réseau étant assemblés de manière à recouvrir la surface d'un plan à peu près perpendiculaire à l'axe du faisceau de radiations, chaque réseau étant décalé angulairement autour de l'axe du faisceau, et des capteurs photoélectriques placés à la périphérie de chaque réseau formée par les extrémités des guides de lumière pour capter les signaux lumineux véhiculés par les guides de lumière, caractérisé en ce qu'il comprend des moyens de séquencement pour commander l'émission de flashs de radiations par la source de radiations en synchronisme avec l'acquisition des signaux par les capteurs photoélectriques.

D'autres caractéristiques et avantages de l'invention apparaîtront ci-après à l'aide de la description qui va suivre faite en regard des dessins annexés qui représentent :

- la figure 1, un mode de réalisation du dispositif selon l'invention ;
- les figures 2, 3 et 4 des modes d'assemblage des capteurs de lumière du dispositif représenté à la figure 1.

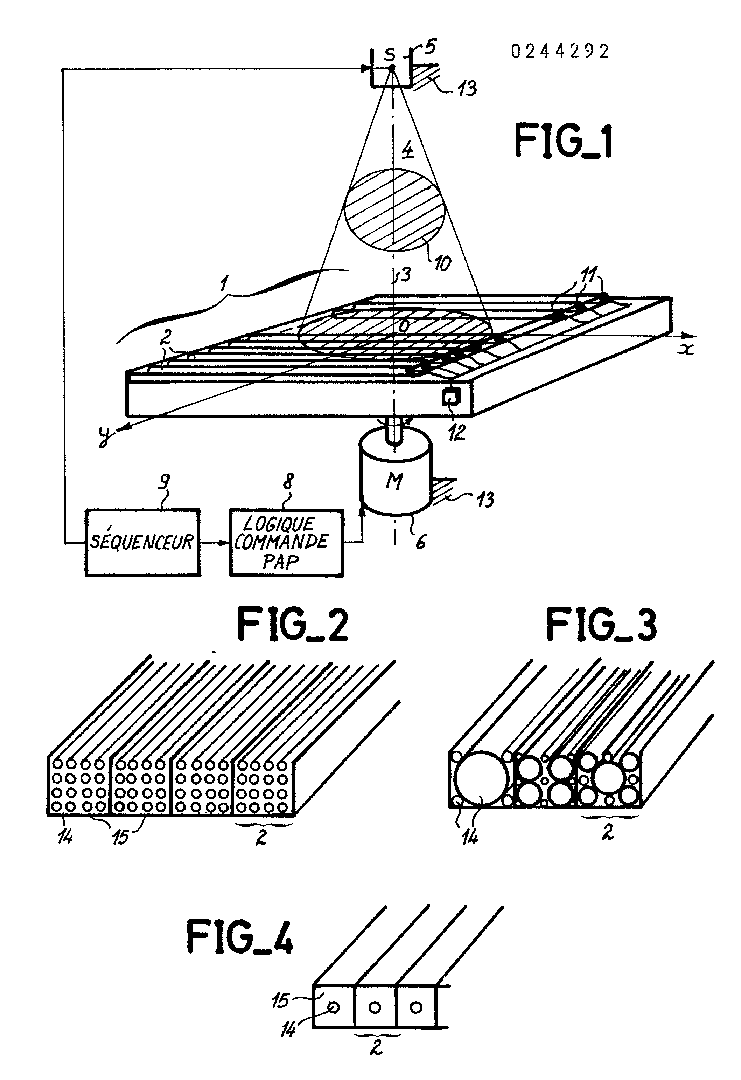

Le dispositif selon l'invention qui est représenté à la figure 1, comprend, un assemblage ou réseau 1 de guides de lumière 2 maintenus parallèles entre eux selon une direction ox dans un même plan perpendiculaire xoy à l'axe 3, d'un faisceau 4 de radiations ionisantes émises par une source de radiation 5 sous la forme, par exemple, d'une source de radiothérapie. Selon des modes de réalisation possibles de l'invention, un des guides de lumière peut être constitué de façon connue par un assemblage de fibres optiques scintillantes, de fibres optiques à transfert de fréquence et de manière générale par tous dispositifs équivalents susceptibles d'émettre à ses extrémités un flux de photons lorsqu'il est irradié par le faisceau 4. L'assemblage 1 de guides de lumière 2 est animé d'un mouvement de rotation autour de l'axe 3 au moyen d'un moteur pas à pas 6 ou tout dispositif équivalent susceptible d'entrainer sous la source 5 un plateau 7 sur lequel est fixé l'assemblage 1 des guides de lumière 2. Le moteur pas à pas 6 est commandé par une logique de commande 8. La source 5 produit des flashes de radiations sous la commande d'un séquenceur 9. Le séquenceur 9 est également relié à la logique de commande 8 pour commander les pas d'avancement du moteur pas à pas 6 en synchronisme avec les flashes de la source 5 et assurer la prise des informations fournies par les guides de lumière en synchronisme avec le déplacement de l'assemblage des guides de lumière, le mouvement de rotation des guides de lumière s'effectuant en l'absence de radiations, le flash de rotation n'étant présent que lorsque ceux-ci sont arrêtés. L'objet 10 irradié dont on veut obtenir l'image des contrastes aux radiations, est interposé entre la source 5 et l'assemblage 1 des guides de lumière. Des capteurs 11 sont placés en regard d'une extrémité de chacun des guides de lumière pour convertir en signaux électriques le signal lumineux reçu pour différentes positions angulaires, ces capteurs 11 étant reliés par un cordon de conducteurs, éventuellement à un connecteur ou prise de courant 12 fixé sur le plateau 7, à des moyens de transmission extérieurs, pour transmettre par tous moyens appropriés non représentés, les signaux obtenus à l'extérieur du dispositif. Sur la figure 1 la source 5 et le moteur 6 sont fixés sur un même chassis 13. Selon un autre mode de réalisation non représenté, les capteurs pourront être fixés directement sur le chassis 13. Dans ce cas, pour des commodités de réalisation le plateau 7 pourra avoir la forme d'un disque ou d'un cylindre, placé à l'intérieur d'une couronne portant sur sa périphérie interne la suite continue des photodiodes 11. La transmission optique peut alors être obtenue directement, au cours du passage des extrémités des guides de lumière devant les photodiodes placées à l'intérieur de la couronne, en synchronisme avec la commande de rotation du moteur pas à pas 6.

Egalement suivant d'autres modes de réalisation de l'invention il sera tout aussi possible de réaliser des assemblages de plusieurs réseaux plans, juxtaposés, de guides de lumière décalés angulairement les uns par rapport aux autres, ce qui pourra avoir l'avantage de réduire, voire supprimer le nombre de décalage angulaire à faire subir aux réseaux.

Un mode d'assemblage de fibres optiques pour former les guides de lumière 2 est représenté à la figure 2. Dans ce mode d'assemblage les guides de lumière sont constitués par des fibres optiques 14 identiques juxtaposées dans l'épaisseur du guide. A titre d'exemple chaque fibre pourra avoir un diamètre de 0,5 mm, un assemblage de $8 \times 8$ fibres pouvant ainsi former une section 15 de guide de 16 mm$^2$.

Par opposition à ce qui existe déjà, le dispositif selon l'invention n'acquiert pas d'informations sur des pixels ou points de l'image mais sur des lignes de l'image. En effet, chacun des guides de lumière, sous l'influence du faisceau de radiations incidentes, émet à ses extrémités un flux de photons, visible ou non, proportionnel au flux de photons incidents. Chaque guide de lumière constitue ainsi une ligne de l'image, et le flux de photons visibles obtenus est proportionnel à la somme des flux de photons X incidents appliqués sur l'ensemble des pixels qui composent la ligne. En négligeant les pertes de transmission du guide de lumière, l'intensité lumineuse produite en sortie d'un guide de lumière peut être représenté par la relation

$$I_s(y) = \int_{-\frac{L}{2}}^{+\frac{L}{2}} \alpha . I(x,y) . dx \qquad (1)$$

$I_s$ étant l'intensité lumineuse obtenue en sortie du guide de lumière d'ordonnée y parallèle à l'axe ox

$I(x,y)$ étant l'intensité lumineuse créée par ionisation de chaque point d'abscisse x dans un guide de lumière d'ordonnée y

L désignant la longueur du guide de lumière

et $\alpha$ étant un coefficient de proportionnalité entre le nombre de photons créés en un point de coordonnées (x,y) et le nombre de photons correspondants en bout du guide de lumière. En tenant compte des pertes de transmission, $\alpha$ est égal à $\alpha(X)$.

En rapprochant la relation (1), de celle connue suivante, donnant la variation d'intensité d'un faisceau de rayons X lors de radiographie par SCANNER

$$\mathrm{Log}\, \frac{I}{I_0} = -\int_{0}^{L} \mu(x,y)\, dl \qquad (2)$$

où $I_0$ désigne l'intensité du faisceau incident, et I désigne l'intensité du faisceau existant au niveau du récepteur situé derrière le patient, on peut constater qu'à condition de faire une acquisition d'informations similaire à celle réalisée pour le SCANNER, les mêmes algorithmes de reconstruction que ceux du SCANNER peuvent être utilisés pour reconstruire l'image, à la différence simplificatrice, cependant, que dans l'invention, la géométrie de l'acquisition est parallèle alors qu'elle est conique pour le SCANNER.

A titre d'exemple, des algorithmes de ce type sont décrits dans les ouvrages suivants :

- BROOKS R. and DICHIRO G., Principles of computer tomography (CAT) in radiographic and radioisotopic imaging, Phys. Med. Biol., 21, 689, 1976 .

- HERMAN, G.T., Image reconstruction from projections : The Fundamental of Computerized Tomography, Academic Press, New York, 1980.

Un mode de fonctionnement d'un dispositif selon l'invention est décrit ci-après dans l'hypothèse où, celui-ci est utilisé dans un appareil de radiothérapie délivrant une dose de 300 rad/mn sous la forme d'impulsions de 5 microsecondes à la fréquence 100 Hz, de photons d'énergie 6 Mev ce qui correspond pour chaque impulsion à une dose

$$D = \frac{300}{60} \cdot \times \frac{1}{100} = 5.10^{-4} \, J/Kg \quad (3)$$

et à un flux de photons $\emptyset_p$ de $1,2810^7$ cm$^{-2}$ ayant traversé un patient, d'épaisseur moyenne 30 cm. En supposant que, d'une part, les guides de lumière sont formés par des fibres scintillantes, de 0,5 mm, constituées essentiellement de carbone de densité voisine de 1 et de pouvoir d'absorption voisin de celui du polystyrène, et que ces guides sont capables de délivrer en leur sortie 12 photons de longueur d'onde $450 \, 10^{-9}$ m lorsqu'ils sont traversés par un électron cobalt de 1,7 MeV et que d'autre part, chaque guide de la lumière a une longueur de 40 cm et une section Se égale à 4 × 4 mm soit 16 cm$^2$ et est réalisé avec un assemblage de 8 × 8 = 64 fibres pour former une ligne d'image avec une résolution de 4 mm, chaque ligne d'image ainsi constituée voit arriver un nombre $N_0$ de photons incidents tel que

$N_0 = \emptyset_p \times S_e$ soit $1,2810^7 \times 16$ photons. (4)

le nombre $N_a$ de photons interagissant avec la fibre vérifie la relation :

$N_a = N_0 \times (1 - EXP(-\mu L))$ (5)

où L est l'épaisseur de la ligne et $\mu$ un coefficient qui dépend du coefficient d'atténuation et du mode d'assemblage des fibres constituant une ligne. En supposant que la ligne est uniformément remplie de fibres, ce qui est le cas si l'on prend des fibres de petit diamètre, l'expression (3) donne pour L = 4mm et $\mu = 2,7410^{-2}$ cm$^{-1}$,

$N_a = 2,23 \times 10^6$ photons d'énergie 6 Mev.

Par analogie avec le comportement de l'eau on doit s'attendre à ce que parmi les photons inter-agissant, 10% créent une paire électron-positron où l'électron a une énergie moyenne de 3,99 Mev et que les autres accélèrent un électron Compton jusqu'à une énergie voisine de 1 MeV.

Comme un électron, traversant 0,5mm de fibre crée 12 photons de longueur d'onde $450 \, 10^{-9}$ m au bout de 40 cm en perdant une énergie de $8,9310^{-2}$ MeV, un électron d'énergie 4 MeV en perdant toute son énergie crée, après 40cm de fibre, $\frac{4}{8,93} \times 10 \times 12$ photons soit 532 photons et un électron d'énergie 1 MeV en crée par conséquent 134.

On peut ainsi obtenir dans ces conditions en bout de ligne un nombre de photons N = $(10^{-1} \times 532 + 910^{-1} \times 134) N_a$ de longueur d'onde $450 \, 10^{-9}$ m sur 16 mm$^2$, en 5 µs.

soit N = $3,88 \times 10^8$ photons.

A titre indicatif, en s'imposant un contraste de 1% par pixel, un dispositif selon l'invention ayant les caractéristiques précédemment indiquées peut, au bout d'une ligne composée par exemple de 100 pixels, détecter une variation relative à $\frac{\Delta N}{N} = 10^{-4}$.

Soit $\Delta Nc = 3,8810^4$ photons

Si d'autre part, on suppose, que les photons de longueur d'onde $450 \, 10^{-9}$m émis par l'absorption des électrons obéissent à une statistique poissonnienne, la fluctuation correspondante s'établit à $\sqrt{N_a} \simeq 1,9710^4$ photons, en-dessous du contraste souhaité. Le rendement en photons très élevé, qui est ainsi obtenu rend possible l'utilisation de simples détecteurs à photodiodes pour la formation des capteurs 11 et à titre indicatif, ces capteurs pourront être constitués par des photodiodes du type connu sous la référence BPW34, commercialisées par la société SIEMENS.

En cours d'exploitation normale le dispositif qui vient d'être décrit peut effectuer des mesures de 5 µs espacées entre elles de 10 ms, chaque mesure fournissant une information sur une barette de 100 capteurs. Ceci rend possible, par exemple, la reconstruction d'une matrice de 128 × 128 points avec 110 mesures pour des positions allant de 0 à 220 degrés au pas de 2 degrés, avec une vitesse de rotation du moteur pas à pas de 0,56 tour/seconde. Des sensibilités par rapport au bruit supérieures à 20 dB peuvent également être obtenues, celles-ci pouvant d'ailleurs être améliorées en disposant par exemple les capteurs photoélectriques 11, non plus que d'un seul côté, mais des deux côtés des guides de lumière, ou en disposant un miroir à l'extrémité opposée de celle où est situé chacun des capteurs.

L'exemple qui vient d'être donné d'un mode préféré de réalisation de l'invention n'est pas limitatif et notamment, en ce qui concerne l'assemblage des guides de lumière, il sera possible d'envisager d'autres types d'assemblage comme le montrent les figures 3 et 4. En effet, à résolution égale plutôt que d'utiliser dans le mode de réalisation de la figure 2 des fibres identiques 14 de diamètre 0,5mm, ce qui en fait 8 × 8 pour une section 15 de ligne de 4 × 4mm$^2$, il pourra être avantageux d'utiliser des fibres 14 de plus gros diamètre, comme le montre la figure 3, combinées avec des fibres de plus petit diamètre pour assurer un remplissage optimal donc une bonne efficacité. Une meilleure efficacité peut encore être obtenue en augmentant l'épaisseur de la ligne, à largeur donc à résolution constante. Cependant le taux de couplage d'une ligne à l'autre risque de s'accroître et il faudra en conséquence blinder de façon connue les lignes entre elles avec un matériau absorbant de photons et réfléchissant les photons visibles. Comme à volume égal, le nombre de photons absorbé par ligne dépend de la densité du matériau absorbant on pourra encore augmenter l'efficacité d'absorption, en utilisant des fibres à coeur liquide comme représenté à la figure 4. Il faudra cependant pondérer cette absorption par une diminution de l'efficacité de conversion électron-photon à la longueur d'onde de $450 \, 10^{-9}$ m.

Encore, selon une autre variante de réalisation, plutôt que d'utiliser des fibres scintillantes il sera tout aussi possible d'utiliser des fibres à transfert de fréquence, noyées dans un scintillateur. Celui-ci, s'il est réalisé par exemple en oxysulfure de Gadolinium peut être beaucoup plus dense et présenter par conséquent une meilleure efficacité. Cependant il ne guide pas la lumière. Pour éviter la diffusion d'une ligne à l'autre il faudra cloisonner les lignes par des miroirs optiques ce qui confinera la lumière. Celle-ci sera ensuite guidée par une ou plusieurs fibres à transfert de fréquence qui absorbera les photons bleus pour émettre et guider les photons verts.

Le principe invoqué ici peut s'appliquer de manière plus générale à toutes les configurations entrant dans les cadres suivants :

1) soit pour recueillir une information à deux dimensions, par exemple, une modulation spatiale d'un faisceau de particules ou de photons, un champ électromagnétique, etc...

2) soit dans le cadre d'un détecteur monodimensionnel, dont la surface sensible est équivalente à une ligne droite, une ligne brisée, courbe etc..., pouvant recueillir une résultante sous la forme d'une somme, somme pondérée, etc... des informations présentes le long de la ligne qu'il occupe dans l'espace.

Alors, dans ces conditions, N détecteurs monodimensionnels, ne se recouvrant pas au sens de la projection selon une direction perpendiculaire à la surface où se trouve l'information à deux dimensions, et placés dans un dispositif autorisant l'ensemble à prendre N positions irréductibles les unes aux autres au sens des algorithmes de reconstruction, permettront toujours, de la façon décrite précédemment, de reconstruire une matrice N × N représentative de l'information à deux dimensions.

## Revendications

1. Dispositif pour l'acquisition d'une image de contraste d'un objet (10) placé dans un faisceau de radiations ionisantes (4) émis par une source de radiations (5), l'objet irradié (10) étant interposé entre la source de radiations et le dispositif, comprenant au moins un réseau de guides de lumière (2) émetteurs de photons sous l'effet des radiations, les guides de lumière (2) d'un réseau étant assemblés de manière à recouvrir la surface d'un plan à peu près perpendiculaire à l'axe du faisceau (3) de radiations, chaque réseau étant décalé angulairement autour de l'axe du faisceau (3), et des capteurs photoélectriques (11) placés à la périphérie de chaque réseau formée par les extrémités des guides de lumière pour capter les signaux lumineux véhiculés par les guides de lumière, caractérisé en ce qu'il comprend des moyens de séquencement (8, 9) pour commander l'émission de flashs de radiations par la source de radiations (4) en synchronisme avec l'acquisition des signaux par les capteurs photoélectriques (11).

2. Dispositif selon la revendication 1, caractérisé en ce que les réseaux de guides de lumière (2) sont empilés dans la direction de l'axe du faisceau (3) et décalés les uns par rapport aux autres autour de l'axe du faisceau (3).

3. Dispositif selon l'une quelconque des revendicatins 1 et 2, caractérisé en ce que chaque réseau a une forme circulaire et est mobile autour de l'axe du faisceau (3) par rapport à une couronne fixe entourant sa périphérie, les capteurs photoélectriques étant disposés à l'intérieur de la couronne et la mise en rotation des réseaux étant assurée par des moyens de mise en rotation par pas angulaires commandés en synchronisme avec l'émission des flashs de radiations, chaque extrémité d'un guide de lumière étant positionné à chaque pas en face d'un capteur photoélectrique.

4. Dispositif selon l'une quelconque des revendications 1 à 3, caractérisé en ce que les guides de lumière sont constitués par des assemblages de fibres optiques scintillantes (14).

5. Dispositif selon l'une quelconque des revendications 1 à 3, caractérisé en ce que les guides de lumière sont constitués par des assemblages de fibres optiques (14) à transfert de fréquence.

6. Dispositif selon la revendication 5, caractérisé en ce que les fibres à transfert de fréquence sont noyées dans un scintillateur réalisé en oxysulfure de Gadolinium.

7. Dispositif selon l'une quelconque des revendications 1 à 3, caractérisé en ce que les guides de lumière (2) sont constitués par des fibres optiques à coeur liquide.

8. Dispositif selon l'une quelconque des revendications précédentes, caractérisé en ce que chaque guide de lumière est constitué par un assemblage de fibres optiques de diamètres différents.

9. Dispositif selon l'une quelconque des revendications 1 à 8, caractérisé en ce que les guides de lumière (2) sont séparés entre eux avec un matériau absorbant de photons et/ou réfléchissant les photons visibles.

10. Dispositif selon la revendication 2, caractérisé en ce que les moyens (6) de mise en rotation pas à pas sont commandés en synchronisme avec les flashes d'une source de radiothérapie.

11. Dispositif selon l'une quelconque des revendications 2, 4 à 10, caractérisé en ce que des capteurs sont disposés à chaque extrémité des guides de lumière.

12. Dispositif selon l'une quelconque des revendications 1 à 11, caractérisé en ce qu'un miroir est placé à l'une des extrémités de chaque guide de lumière (2).

0244292

# FIG_1

SÉQUENCEUR

LOGIQUE COMMANDE PAP

# FIG_2

# FIG_3

# FIG_4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | GB-A-2 061 055  (EMI LTD) <br> * Abrégé; page 1, lignes 72-84,98 - page 2, lignes 2,8-98;  figures * | 1,4,9 | G 01 T   1/29 <br> G 01 T   1/164 <br> G 01 T   5/08 <br> A 61 B   6/03 |
| A | | 3,12 | |
| Y | FR-A-2 425 082  (GALILEO ELECTRO-OPTICS CORP.) <br> * Page 4, ligne 26 - page 5, ligne 25; page 7, lignes 4-11; page 10, ligne 15 - page 11, ligne 10; page 4, lignes 2-17; revendication 1; figures * | 1,4,9 | |
| A | FR-A-2 555 321  (CENTRE NATIONAL DE LA RECHERCHE SCIENTIFIC) <br> * Page 2, ligne 15 - page 3, ligne 2; figures * | 4,7,9, 12 | **DOMAINES TECHNIQUES RECHERCHES (Int. Cl.4)** <br><br> G 01 T <br> A 61 B |
| A | PATENT ABSTRACTS OF JAPAN, vol. 9, no. 334 (P-417)[2057], 27 décembre 1985; & JP-A-60 159 675 (SHIMAZU SEISAKUSHA K.K.) 21-08-85 | 4,9,11 | |

Le présent rapport de recherche a été établi pour toutes les revendications

| Lieu de la recherche | Date d'achèvement de la recherche | Examinateur |
|---|---|---|
| LA HAYE | 04-08-1987 | DATTA S. |